(19)

Europäisches
Patentamt
European
Patent Office
Office européen
des brevets

(11) **EP 4 056 037 A1**

(12) **EUROPEAN PATENT APPLICATION**
published in accordance with Art. 153(4) EPC

(43) Date of publication:
**14.09.2022 Bulletin 2022/37**

(21) Application number: **20880730.5**

(22) Date of filing: **28.10.2020**

(51) International Patent Classification (IPC):
**A01N 25/22** (2006.01)  **A01N 25/30** (2006.01)
**A01N 55/02** (2006.01)  **A01N 55/10** (2006.01)
**A01N 59/06** (2006.01)  **A01N 59/16** (2006.01)
**A01P 1/00** (2006.01)  **A01P 3/00** (2006.01)

(52) Cooperative Patent Classification (CPC):
**A01N 25/02; A01N 25/22; A01N 25/30;**
**A01N 35/00; A01N 55/02; A01N 59/06;**
**A01N 59/16; A61K 9/50**

(86) International application number:
**PCT/IB2020/060115**

(87) International publication number:
**WO 2021/084448 (06.05.2021 Gazette 2021/18)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **29.10.2019  CO 19012045**

(71) Applicant: **Biológicos Estratégicos Bioest S.A.S.**
**Siberia, Cundinamarca (CO)**

(72) Inventor: **BLANCO TIRADO, Cristian**
**Piedecuesta, Santander (CO)**

(74) Representative: **ABG Intellectual Property Law,**
**S.L.**
**Avenida de Burgos, 16D**
**Edificio Euromor**
**28036 Madrid (ES)**

(54) **GREEN ANTIFUNGAL AGROCHEMICAL FORMULATION BASED ON NANOMATERIALS OF SILICON AND ZINC**

(57)    The present invention relates to a stable agrochemical formulation, comprising a silicate salt, zinc oxide nanoparticles, a vegetable oil, a surfactant or a mixture of surfactants, a dispersant or a mixture of dispersants and, a stabilizer or mixture of stabilizers useful in the prevention or control of a plant disease produced by a microorganism, such as fungi or oomycetes. Additionally, the present invention relates to a method for preparing such agrochemical formulations. The agrochemical formulations and the method of the invention facilitate the preparation of agrochemical formulations containing silicate salts and zinc oxide nanoparticles, which furthermore decrease the environmental impact produced by the dispersion of pesticidal, bactericidal, fungicidal and oomyceticidal compounds.

EP 4 056 037 A1

**Description**

**FIELD OF INVENTION**

The present invention belongs to the field of agricultural chemistry, and in particular, to agrochemical formulations designed for the physical and chemical protection of different plants and crops to mitigate the incidence of pathogenic fungi in agricultural production.

**BACKGROUND OF THE INVENTION**

It is estimated that by 2050 there will be a population of 9.4 billion people, which implies increasing food production by about 50% ("Solar Energy Supply and Storage for the Legacy and Nonlegacy Worlds", Timothy R. Cook et al., Chem. Rev. 2010, 110, 6474-6502; "Can we improve global food security? A socio-economic and political perspective", Ulrike G., Food Sec. 2014, 6, 187-200). However, producing 50% more food requires land suitable for cultivation, a permanent supply of water, seeds, fertilizers and mechanisms for pest control, specifically insects and fungi. Considering that annually 10-16% of global crops are lost due to plant diseases, with losses of about 220 billion dollars ("Climate change, plant diseases and food security: an overview", Sukumar C. et al., Plant Pathology. 2011, 60, 2-14), it is evident that there is an urgent need to create a new generation of pesticides (insecticides and fungicides) with particular features such as: (i) high availability of non-toxic raw materials, (ii) easy production, (iii) high specificity against families of organisms, (iv) harmlessness of the substances comprising the fungicide for humans, mammals and noble insects, e.g., bees, and (v) low environmental footprint in the fungicide synthesis, among others.

Dithiocarbamates are one of the most widely used types of synthetic organic chemicals in the prevention and control of fungal plant diseases. Commercial fungicides formulated with these substances are widely used. However, this class of fungicides has disadvantages such as their synthesis footprint, the production complexity and the active substance biotoxicity, which does not meet several of the desirable aforesaid characteristics.

Usually, active compounds in fungicides have high efficiency and efficacy in controlled laboratory assays. However, it is well known that for the use of these materials in the field it is required to create a formulation including adjuvants, binders, dispersants, defoamers and other molecules that improve its application.

Different strategies are used to formulate products used for the control of pathogenic microorganisms. For example, US letters patent 8,404,263 discloses an agrochemical formulation containing a pesticide, an organic UV photoprotective filter and metal oxide coated nanoparticles. The objective of the invention disclosed therein is to provide a composition that decreases the degradation of pesticidal compounds by their exposure to sunlight, especially UV.

On the other hand, US patent application 2010/0016443 discloses a method for the preparation of particles of a pesticide compound comprising a metal oxide coating and compositions comprising such particles. The invention described therein allows the isolation of the active compound (pesticide) allowing furthermore a controlled release thereof.

Additionally, patent applications EP0496106 and MX2017012740 disclose stable agrochemical compositions comprising an α-unsaturated amine derivative, salts thereof or a ferrous sulfate compound and liquid paraffin respectively, which act as fungicidal insecticides to prevent pests in plants and/or crops.

Considering the foregoing it may be concluded that, in general, commercial fungicide formulations contain a wide variety of organic adjuvant, fixative and dispersant molecules, among others. The function of these molecules is to improve the product application and to make the active molecules fix in a prolonged way to the plant creating a residual effect. Since fungicides are applied by foliar spraying, the residual effect is transferred to the fruits and leaves for human consumption. Therefore, the new generations of fungicides must include in their formulation chemical substances that have a proven selectivity towards pathogens and harmlessness towards beneficial animals in crops, small mammals and humans. It is also sought that these substances do not contaminate soils and water sources.

Recent studies show that silicon has several functions in plant physiology, mainly in grasses. Among the physiological functions are tolerance to toxicity caused by heavy metals, mechanical protection against pathogens and, in some cases, it is even considered as an essential nutrient that contributes to plant growth ("Silicon and Plan Diseases", Fabricio R. et al., Springer. 2015). In fact, according to "Benefits of plant silicon for crops: a review", Guntzer F, Agron Sustain Dev. 2012 32,201-213, seven of the ten most produced crops in the world are silicon accumulators, including corn, rice, sugar beet, sugar cane and wheat.

Silicon has also been used to prevent and control diseases in different crops through different strategies, for example, in the control of soil-borne and seed-borne diseases. Thus, according to the review elaborated by Alessandro F. in "Silicon and Plan Diseases", Fabricio R. et al., Springer, Chapter 3, 2015, supplementing plant nutrition with silicon reduces the effect of certain diseases in avocado, banana, red pepper, coffee, corn, cucumber, lettuce, melon, rice, tomato, watermelon, wheat, among others, attacked by the pathogens *Phytophthora cinnamomi, Cylindrocladium spathiphylli, Fusarium oxysporum* sp. *cubense, Meloidogyne javanica, Phytophthora capsici, Meloidogyne exigua, Pythium aphanidermatum, Fusarium moniliforme, Pythium ultimum, Pythium aphanidermatum* sp. *cucumerinum, Fusarium*

*oxysporum* sp. *lactucae, Fusarium* spp., *Meloidogyne* spp. Silicates also help to control leaf diseases of monocotyledonous and dicotyledonous plants, caused by fungi, bacteria and viruses.

On the other hand, according to "Benefits of plant silicon for crops: a review", Guntzer F, Agron Sustain Dev. 2012 32,201-213, the benefits of silicon in plants have been more easily evidenced under environmental stress. These benefits are summarized as increased resistance to pathogens and insects, relief during drought, reduced incidence of stress from high salt concentrations, improved uptake of potassium, phosphorus and calcium, regulation of nutrient uptake (phosphorus and nitrogen) when in excess, and alleviation of aluminum and zinc toxicity. In particular, silicon also provides mechanical benefits to the plant derived from the presence of silicon phytoliths in the organic tissue, which provide increased resistance to strong winds and rain, as well as increased resistance to insects.

One of the ways to incorporate silicon in the treatment of crops is by spraying silicate solutions on plants, because they form a physical barrier on the surface of the plant that prevents pathogens from infecting it. Recently it has been shown that silicates allow the activation of certain defense pathways in plants against pathogens ("*Silicon and Plan Diseases*", Fabricio R. *et al.,* Springer, 2015). Due to their harmlessness, silicates do not present the environmental problems of other agrochemicals and pesticides, because their life cycle has not been shown to be toxic ("Soluble Silicates-Highly Versatile and Safe", CH. Baehr et al., International Journal for Applied Science, 2007, 133, 88-94). In addition, silicates promote plant health and prevent microorganisms or other pests from opportunistically attacking plants.

However, although silicates have certain advantages as useful substances, such as agrochemicals, mixtures containing silicates are unstable because they precipitate at pH values below 9. For this reason, formulations that use silicates as an active ingredient are marketed in the form of Na or K silicates that show pH values higher than 9. For example, Sil-MATRIX® is a potassium silicate formulation that serves as a preventive fungicide, recommended for agricultural crops, fruits, nuts, among others, requires dilution prior to spraying on the plant, where continuous agitation is recommended to obtain a thorough mixture and avoid storage and mixing of the diluted material (http://www.certisusa.com/pdf-labels/Sil-Matrix label.pdf). In addition to this disadvantage, there is another aspect related to the low foliar fixation capacity of silicates in liquid state, which implies administering larger doses with permanent repetitions to guarantee the expected effects on crops.

On the other hand, recent studies also show that the use of nanomaterials, specifically nanoparticles of transition metal oxides, have great reactivity in various physical and chemical processes. The reactivity of these materials is based on their large surface area, which allows greater contact area of the nanoparticulated material with molecules or microorganisms in a solution or surface. For example, silver (Ag) is a very effective bactericide, because it is capable of coupling to molecules that have double bonds, especially those found in the cell wall. The formation of such silver nanoparticle/cell wall complexes promotes exposure of the cytoplasm and consequent cell death.

Several authors have demonstrated, under controlled conditions in the laboratory, that zinc oxide nanoparticles also produce the opening of the cell wall in bacteria of the *Campylobacter* ("Antibacterial Activity and Mechanism of Action of Zinc Oxide Nanoparticles against Campylobacter jejuni", Yanping X, et al., Journal of American Society for Microbiology, 2011; 77(7): 2325-2331). On the other hand, other authors have shown that zinc oxide nanoparticles in conjunction with graphene also act as bactericides (Synthesis, characterization and enhanced antimicrobial activity of reduced graphene oxide-zinc oxide nanocomposite", Rajveer S, et al., Mater. Res. Express, 2017,1-8), while Lili He *et al. (2011)* developed assays showing that zinc oxide nanoparticles have antifungal properties against *Botrytis* and *Penicillinum* (Lili He., Yang Liu., Azlin Mustapha., Mengshi Lin., Microbiological Research. 2011, 166, 207-215). These studies also demonstrate that the action mechanism of zinc oxide nanoparticles could be controlled by cell wall modification, which promotes a shape and size distortion of fungal and bacterial cells, when in contact with the nanoparticulate material, eventually causing cell death by wall opening.

Despite the potential of zinc oxide nanoparticles as a bactericide and fungicide, the stabilization and formulation of this nanomaterial represents a technological challenge.

The present invention describes the preparation of a stable agrochemical formulation comprising a silicate salt and zinc oxide nanoparticles, together with a vegetable oil, a surfactant or a mixture of surfactants, a dispersant or a mixture of dispersants and a stabilizer or mixture of stabilizers. The agrochemical formulations containing silicates of the present invention are characterized by preventing and remedying the problems associated with plant diseases caused by phytopathogenic fungi, and also uses non-toxic chemical substances of organic nature and harmless to humans and beneficial organisms, in its production, which generates little environmental impact.

## BRIEF DESCRIPTION OF THE INVENTION

The present invention corresponds to an agrochemical formulation comprising a silicate salt, a vegetable oil, a surfactant or a mixture of surfactants, a dispersant or a mixture of dispersants, zinc oxide nanoparticles and a stabilizer or mixture of stabilizers, as well as methods for preparing the same. Particularly, the formulation is characterized in that it is stable for long periods of time and it is useful in the prevention and control of plant diseases caused by microorganisms such as fungi or oomycetes. Therefore, the agrochemical formulation has application in the agricultural industry, as a natural or organic fungicide with low environmental impact.

## BRIEF DESCRIPTION OF THE FIGURES

**FIG. 1** Powder X-ray diffractogram of **(A)** ZnO nanoparticles synthesized via Sol-Gel (Example 1), **(B)** dry-synthesized ZnO nanoparticles (Example 2) and **(C)** commercial USP ZnO nanoparticles.

**FIG. 2** FT-IR spectra of ZnO nanoparticles: **(A)** Sol-Gel synthesis (Example 1) and **(B)** dry synthesis (Example 2).

**FIG. 3** UV-VIS spectra of zinc oxide nanoparticles **(A)** dry synthesis of solvent $H_2O$, **(B)** Sol-Gel synthesis of solvent $H_2O$, **(C)** Sol-Gel synthesis of solvent PEP- $H_2O$ and **(D)** Sol-Gel synthesis of solvent PEP-$CH_3H_8O$.

**FIG. 4** Scanning electron microscopy (SEM): (A) Dry-synthesized zinc oxide nanoparticles; **(B)** Sol-Gel-synthesized zinc oxide nanoparticles; **(C).** Commercial USP zinc oxide nanoparticles.

**FIG. 5** Scanning electron microscopy (SEM) for agrochemical formulations of Example 7: **(A)** Agrochemical formulation D; **(B)** Agrochemical formulation F and **(C).** Agrochemical formulation H.

**FIG. 6** UV-VIS spectra of agrochemical formulations of Example 7 (for 10 days) **(A)** Agrochemical formulation D and **(B)** Agrochemical formulation H.

**FIG. 7** Stability curve of the agrochemical formulations of Example 7 **(A)** Agrochemical formulation D, **(B)** Agrochemical formulation F and **(C)** Agrochemical formulation H.

**FIG. 8** Antifungal activity of the agrochemical formulations of Example 7 against *B. cinérea* based on time: **(A)** Agrochemical formulation D1, **(B)** Agrochemical formulation F1, **(C)** Agrochemical formulation H1, **(D)** Agrochemical formulation I, (E) Agrochemical formulation J.

**FIG. 9** Antifungal activity of the agrochemical formulations of Example 7 against *M. fijiensis* based on time: **(A)** Agrochemical formulation D1, **(B)** Agrochemical formulation F1, **(C)** Agrochemical formulation H1, **(D)** Agrochemical formulation I, (E) Agrochemical formulation J.

**FIG. 10** Antifungal activity of the agrochemical formulations of Example 7 against *P. palmivora* based on time: **(A)** Agrochemical formulation D, **(B)** Agrochemical formulation F, **(C)** Agrochemical formulation H, **(D)** Agrochemical formulation I, **(E)** Agrochemical formulation J.

**FIG. 11** Antifungal activity of the agrochemical formulations of Example 7 against *P. infestans* based on time: **(A)** Agrochemical formulation D, **(B)** Agrochemical formulation F, **(C)** Agrochemical formulation H, **(D)** Agrochemical formulation I, **(E)** Agrochemical formulation J.

**FIG. 12** Antifungal activity based on time with the commercial fungicides: Siganex (C1), Mancozed 80% WP (C2) and Forum 500 WP (C3) against; **(A)** B. *cinerea*, **(B).** *M. fijiensis,* **(C)** *P. palmivora* and **(D)** *P. infestans.*

## DETAILED DESCRIPTION

The present invention corresponds to an agrochemical formulation comprising a silicate salt and zinc oxide nanoparticles in a liquid matrix, containing vegetable oil, a surfactant or a mixture of surfactants, a dispersant or a mixture of dispersants and a stabilizer or mixture of stabilizers, as well as methods for their preparation. In particular, this invention provides an agrochemical formulation comprising a silicate salt and zinc oxide nanoparticles that are stable over time.

For the purposes of the present application, the terms *"silicate"* or *"silicate salt"* refer to a salt comprising the $(SiO_3)^{2-}$ anion and any cation, including but not limited to sodium, potassium, manganese, magnesium and calcium. In one embodiment of the invention, the silicate or silicate salt is selected from potassium silicate, sodium silicate, manganese silicate, magnesium silicate and calcium silicate.

The silicate salt is contained in the agrochemical formulation of the invention in a concentration between 0.01 and 250 g/L. In another embodiment of the invention, the concentration is selected between 0.05 and 240 g/L, 0.1 and 230 g/L, 0.5 and 220 g/L, 0.8 and 210 g/L, 1.0 and 200 g/L, 1.5 and 190 g/L, 2.0 and 180 g/L, 2.5 and 170 g/L, 3.0 and 160 g/L, 3.5 and 150 g/L, 4.0 and 140 g/L, 4.5 and 130 g/L, 5.0 and 120 g/L, 5.5 and 110 g/L, 6.0 and 100 g/L, 6.5 and 95 g/L, 7.0 and 90 g/L, 8.5 and 85 g/L, 9.0 and 80 g/L, 10 and 75 g/L, 15 and 70 g/L, 20 and 65 g/L, 25 and 60 g/L, 30 and 55 g/L, 35 and 50 g/L, and 40 and 45 g/L.

Additionally, the term *"vegetable oil"* refers to a viscous, liquid mixture at room temperature extracted from a plant and made of non-polar compounds, mainly triglycerides. Possible vegetable oils include, but are not limited to soybean oil, corn oil, cottonseed oil, olive oil, palm oil, peanut oil, canola oil, safflower oil, sesame oil, sunflower oil, hazelnut oil, almond oil, walnut oil, macadamia oil, pecan oil and pistachio oil. In one embodiment of the invention, the vegetable oil is selected from the group consisting of soybean oil, corn oil, cottonseed oil, olive oil, palm oil, peanut oil, canola oil, safflower oil, sesame oil and sunflower oil. Vegetable oil is found at a concentration between 0.001 to 5.0% v/v, 0.01 to 3.0% v/v and 0.1 to 1.0% v/v.

Additionally, the term *"surfactant"* used in the present application refers to a compound which comprises polar and non-polar groups and decreases the surface tension of a liquid or the interfacial tension of two immiscible substances. Synonyms thereof are *"surfactant"* and "*surface-active agent*". The surfactant comprising the agrochemical formulation of the invention is selected from ionic and non-ionic surfactants. In one embodiment of the invention, the surfactant is non-ionic. For example, the non-ionic surfactant is selected from, but not limited to, the group comprising ethoxylated linear alcohols, ethoxylated alkylphenols, fatty acid esters, amine derivatives, amide derivatives, alkylpolyglucosides, ethylene glycol/propylene glycol copolymers, polyols, ethoxylated polyols, thiols (mercaptans) and derivatives thereof or a mixture thereof. Particularly, the non-ionic surfactant is selected, but not limited to, the group comprising polysorbate, poloxamer, octylglycoside, polyglycerol, polyricinoleate, Triton X-100, cetyl alcohol and Cirrasol. Especially, it may be polysorbate 20 (Tween 20), polysorbate 40 (Tween 40), polysorbate 60 (Tween 60), polysorbate 65 (Tween 65), polysorbate 80 (Tween 80), Cirrasol or a mixture thereof. The surfactant or mixture of surfactants is at a concentration between 0.0001 to 1% v/v, 0.001 to 0.1% v/v, and 0.001 to 0.01% v/v.

As used in the present application, the term *"thickener"* refers to a compound that increases the viscosity of a liquid. A synonym for this term is *"viscosifier"*. The thickener or viscosifier comprising the formulation of the present invention is an organic, polysaccharide, protein, thickener or viscosifier, a derivative thereof or a mixture thereof. For example, polyvinyl alcohol, methylcellulose, xanthan gum, hydroxymethylcellulose, gum arabic, carboxymethylcellulose, hydroxyethylcellulose, hydroxypropylcellulose, hydroxypropylmethylcellulose, gellan gum, guar gum, locust bean gum, tragacanth gum, succinoglucan gum, gelatin, carrageenan, starch, sago, tapioca, pectin, collagen and agar. In particular, the thickener or viscosifier is a polysaccharide, such as methylcellulose, xanthan gum, hydroxymethylcellulose, gum arabic, carboxymethylcellulose, hydroxyethylcellulose, hydroxypropylcellulose, hydroxypropylmethylcellulose, gellan gum, guar gum, locust bean gum, tragacanth gum, succinoglucan gum, gelatin, carrageenan, starch, agar. The thickener is at a concentration between 0.0001 to 10.0 g/L, 0.001 to 8.0 g/L, 0.01 to 6.0 g/L, 0, y1 to 4.0 g/L.

As used herein, the term "*nanofluid*" refers to a fluid containing uniformly and stably suspended nanoparticles.

As used in the present invention, the term *"stable"* refers to the formulation containing silicates and zinc oxide nanoparticles remaining homogeneous, without separation of its components or phases for a storage time exceeding 30 days.

In one embodiment of the invention, the agrochemical formulation is in the form of an emulsion, consisting of a two-phase system, in which one of the non-miscible liquids is uniformly dispersed in the other. In the present invention, the two phases are formed with the silicate and the vegetable oil, together with the surfactant. This emulsion also comprises zinc oxide nanoparticles. As used in the present application, the term *"nanoparticles"* refers to a particle with a size between 1 and 999 nm.

In one embodiment of the present invention, the agrochemical formulation comprises a silicate, a vegetable oil, a surfactant or a mixture of surfactants, a dispersant or a mixture of dispersants, a stabilizer or mixture of stabilizers and zinc oxide nanoparticles. The zinc oxide nanoparticles are in a concentration between 0.1 and 300 g/L. In one embodiment of the invention, the zinc oxide nanoparticles have a size of 1 to 100 nm, 10 to 80 nm, 25 to 80 nm and 25 to 50 nm.

In a further embodiment of the invention, the agrochemical formulation comprises a silicate salt, a vegetable oil, a surfactant or a mixture of surfactants, a stabilizer or mixture of stabilizers, zinc oxide nanoparticles, further comprising a dispersant or a mixture of dispersants. As used in the present application, the term *"dispersant"* refers to a substance that allows the zinc oxide nanoparticles to remain dispersed in solution preventing their aggregation. In one embodiment of the invention, the dispersant is selected from a mixture of ammonium citrate and glycerol (in a 1:1.5 ratio) and sodium hexametaphosphate. The dispersant is in a concentration between 0.1 to 500 g/L, 0.5 to 400 g/L, 1.0 to 300 g/L, 5.0 to 200 g/L, 10.0 g/L to 100 g/L, and between 0.1 to 40 g/L.

The zinc oxide nanoparticles incorporated in the agrochemical formulations of the present invention may be synthesized by any technique known to a person averagely versed in the matter, such as the colloidal method, photochemical and radiochemical reduction, microwave irradiation, use of dendrimers, solvothermal synthesis, sol-gel synthesis, dry synthesis, among others. In one embodiment of the invention, zinc nanoparticles are synthesized by modifications of the Sol-Gel methodology developed by "Synthesis and characterization of zinc oxide nanoparticles: application to textiles as UV-absorbers", Becheri A., et al., J. Nanopart. Res, 10: 679-689 and by the methodology developed by "Low temperature synthesis of ZnO nanoparticles using mechanochemical route: a green chemistry approach" Azam A et al., IJTAS, 2009, 1(2): 12-14.

The preparation of the foregoing agrochemical formulations is carried out through the following methods, corresponding to invention modalities. The formulation comprising a silicate salt, a vegetable oil and a surfactant or a mixture of surfactants, a dispersant or a mixture of dispersants, zinc oxide nanoparticles, and a stabilizer or mixture of stabilizers is made according to a method comprising the steps of:

a) mixing an aqueous solution of the silicate salt with a vegetable oil;
b) adding a surfactant or a mixture of surfactants;
c) adding a stabilizer or a mixture of stabilizers;
d) preparing a nanofluid comprising zinc oxide nanoparticles and a dispersant;
e) mixing the product resulting from step a) with the nanofluid from step d).

The formulations of the invention are directed to use for the prevention or control of a plant disease produced by a microorganism. For example, the plants may be vegetables, plants producing fruits or infructescences, ornamental plants, medicinal plants, legumes, grains and tubers. In particular, crops of chard (*Beta vulgaris*), peppers (*Capsicum spp.*), garlic and onions (*Allium spp.* ), celery *(Celery gravolens),* eggplant (*Solanum meolongena*), pumpkin (*Curcurbita moschata*), chayote *(Sechium edule),* cabbage (*Brassica oleracea*), spinach (*Spinaca oleracea*), beans *(Phaseolus vulgaris),* lettuce (*Lactuca sativa*), maize (*Zea mays*), peanut (*Arachis hypogaea*), tomato (*Solanum lycopersicum),* cucumber *(Cucumis sativus),* okra *(Hibiscus esculentus),* radish *(Raphanus sativus),* beetroot (*Beta vulgaris),* carrot (*Daucus carota*), avocado *(Persea auericana)*, anon *(Annona squamosa)*, caimito (*Chrysophyllum cainito*), canistel (*Pouteria campechiana*), cherry (*Malpighia punicifolia*), custard apple (*Annona reticulata*), plum (*Spondias dulcis),* coconut (*Coco nucifera*), papaya (*Carica papaya*), soursop (*Annona muricata*), guava (*Psidium guajaba*), pomegranate *(Punica granatum),* lime (*Citrus aurantifolium*), lemon *(Citrus limonum),* red mamey (*Calocarpum mammosum*), mamey santo domingo (*Mammea americano*), mamoncillo (*Melicocea bijuga*), mandarin (*Citrus reticulata*), mango (*Mangifera indica*), passion fruit *(Passiflora laurifolia)*, water melon *(Citrullus vulgaris),* sour orange (*Citrus aurantium*), sweet orange *(Citrus sinensis),* pineapple *(Ananas comosus),* banana *(Musa paradisiaca),* plantain (*Musa balsisiana*), tamarind (*Tamarindus indica*), grapefruit *(Citrus paradisi),* Creole grapefruit (*Citrus grandin*), grape (*Vitis vinifera*), bean *(Phaseolus vulgaris),* maize (*Zea mays),* rice (*Oryza sativa*), coffee (*Coffea arabica*), sugar cane *(Saccharum officinarum),* cotton *(Gossypium hirsutum),* gherkin (*Melothria guadalupensis*), sweet potato *(Ipomoes batatas),* potato (*Solanum tuberosum),* creole potato (*Solanum phureja*), cape gooseberry (*Physalis peruviana*), cassava (*Manihot esculenta*), soybean *(Glycine max*), strawberry (*Fragaria spp.* ), blackberry *(Morus* spp.), bramble *(Rubus* spp.), palms of the Aracaceae family, oil palm (*Elaeis guineensis),* cocoa (*Theobroma cacao*), tree tomato (*Solanum betaceum),* lulo (*Solanum quitoense).*

In particular, the microorganism is a bacterium, fungus or oomycete. For example, the bacterium is *Pseudomonas syringae, Ralstonia solanacearum, Agrobacterium tumefaciens, Xanthomonas* spp. (including but not limited to *Xanthomonas oryzae* pv. oryzae, *Xanthomonas campestris, Xanthomonas axonopodis, Erwinia amylovora, Xylella fastidiosa, Dickeya dadantii, Dickeya solani, Pectobacterium carotovorum, Pectobacterium atrosepticum, Clavibacter michiganensis, Clavibacter sepedonicus, Pseudomonas savastanoi.* In particular, the fungi are *Magnaporthe oryzae, Botrytis cinerea, Puccinia* spp. (including but not limited to *Fusarium graminearum, Fusarium oxysporum), Blumeria graminis, Mycosphaerella* spp. (including but not limited to *Mycosphaerella fijiensis* and *Mycosphaerella graminicola), Colletotrichum* spp., *Ustilago maydis, Melampsora lini, Phakopsora pachyrhizi* and *Rhizoctonia solani.* As for the oomycete, this may be *Phytophthora* spp. (including but not limited to *Phytophthora infestans, Phytophthora ramorum, Phytophthora sojae, Phytophthora capsici, Phytophthora cinnamomi, Phytophthora palmivora* and *Phytophthora parasitica, Hyaloperonospora arabidopsidis, Plasmopara vitícola, Pythium ultimum, Albugo candida* and *Peronosporafarinosa).*

## EXAMPLES

### Example 1: Sol-Gel synthesis of ZnO nanoparticles

An amount of 5.5 g of $ZnCl_2$ was dissolved in 200 mL of water at 90°C in an oil bath. Then, 16 mL of 5 M of NaOH was added dropwise to the $ZnCl_2$ solution with gentle stirring over a period of 10 minutes at 90°C. The particles were separated from the supernatant dispersion by sedimentation and, then, the supernatant solution was discarded. The suspension was washed five times with distilled water to reduce the NaCl concentration to less than 1 $\mu M$ by measuring the removal of NaCl from the suspension through $AgNO_3$ solution. The obtained purified particles were peptized with 2-propanol in an ultrasonic bath for 10 min to disintegrate the micro-agglomerates and obtain ZnO nanounits. Then, the particles were separated by centrifugation at 6000 rpm for 15 min. The washing procedure was carried out three times. Finally, the particles were subjected to 250°C for 5 hours to obtain 1.5 g of zinc oxide nanoparticles.

**Example 2: Dry Synthesis of ZnO nanoparticles**

An amount of 21.9 g of zinc acetate and 18 g of tartaric acid were mixed by grinding in an agate mortar for 30 min at room temperature to produce zinc tartrate. The zinc tartrate was subjected to 450°C for one hour to obtain 7 g of zinc oxide nanoparticles.

**Example 3: X-ray powder diffraction characterization of ZnO nanoparticles**

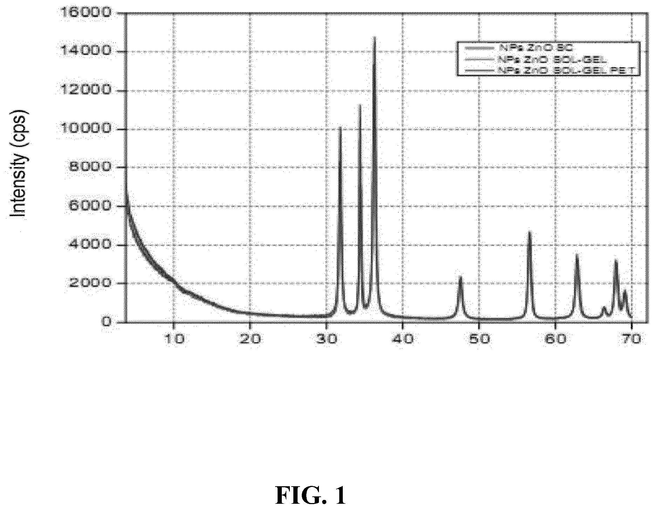

As seen in FIG. 1A and 1B, the zinc oxide nanoparticles synthesized according to Example 1 and 2 present a crystal structure corresponding to the hexagonal wurtzite phase and with similar lattice parameters (a=3.251 y c=5.205 Å). No additional phase is observed, assuring the purity of the obtained compound. Particularly the diffractograms obtained from the synthesized nanoparticles present the same peaks of the commercial USP nanoparticles (FIG. 1C).

**Example 4: Characterization by FT-IR Spectroscopy of ZnO nanoparticles**

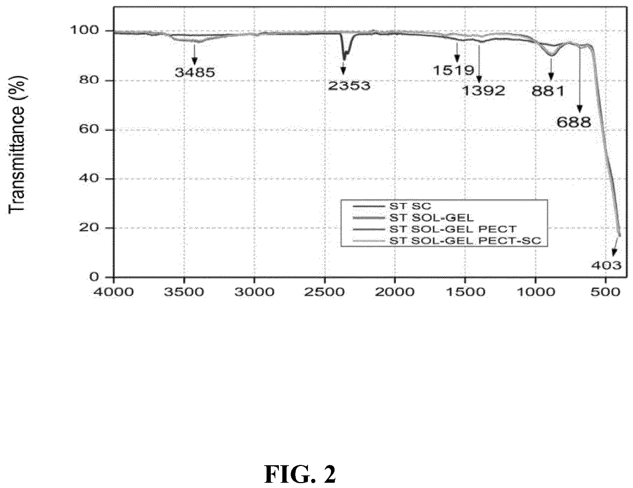

The FT-IR spectra of the zinc oxide nanoparticles synthesized according to Example 1 and 2 (FIG 2A and 2B) show the Zn-O absorption band near 430 $cm^{-1}$. The peaks at 3450 and 2350 $cm^{-1}$ indicate the presence of -OH and C=O traces, probably due to the moisture and $CO_2$ present in the atmosphere. The same spectrum was obtained from the nanoparticles produced by the following dry synthesis (ST SC), sol-gel synthesis (ST SOL-GEL), sol-gel synthesis with 2-propanol pectization (ST SOL-GEL PECT), sol-gel synthesis with 2-propanol pectization and oven drying at 120°C (ST SOL-GEL PECT SC).

It is interpreted in the FT-IR spectrum the presence of zinc oxide with the intense band in the wavelength of 400 $cm^{-1}$ in the ZnO NPs by dry synthesis of analytical precursors (ZnO NPs SS Analit), using the same synthesis and changing the precursor tartaric acid by the commercial one (ZnO NPs SS AT) presents similar spectrum and the compound ZnO USP also showed the same spectrum.

**Example 5: UV-Vis Spectroscopy Characterization of ZnO nanoparticles**

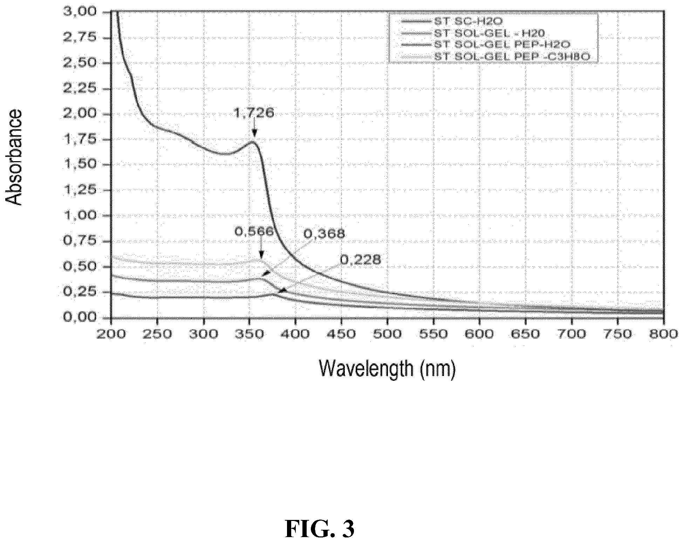

The UV-Vis spectra of zinc oxide nanoparticles synthesized according to Example 1 and 2 using different solvents are shown in FIG. 3A, 3B, 3C and 3D. The absorbance is presented from 372 and 362 nm for aqueous and organic. The highest absorbance of 1.72 for the sol-gel synthesis with pectization dissolved in deionized water (ST SOL-GEL PECT-H2O), the intermediate magnitudes 0.36 - 0.56 corresponding to: sol-gel synthesis dissolved in distilled water (ST SOL-GEL-H2O), propanol dissolved in 2-propanol (ST SOL-GEL PECT-C3H8O, and the lower magnitude 0.228 corresponding to dry synthesis dissolved in deionized water (ST SC-H2O), the latter value is also reported by the authors [4, 6], typical of ZnO NPs.

By UV-visible spectroscopy the zinc oxide is reported with wavelength in the range of 370-390 nm with an absorbance percentage between 0.22% to 1.80% allowing to identify, evaluate qualitatively the stability and purity of the compound.

**Example 6: Scanning Electron Microscopy (SEM) characterization of ZnO nanoparticles.**

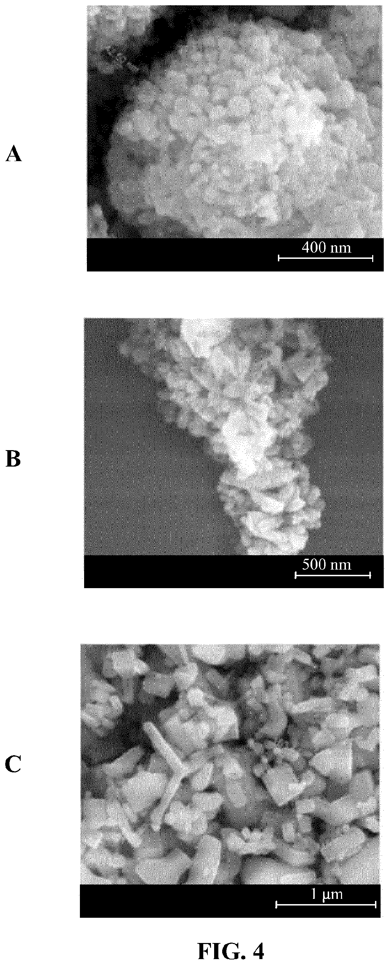

As seen in Figure 4, the zinc oxide nanoparticles in Example 2 (Figure 4A) are spherical and have diameters between 25 and 47.2 nm or are irregular and form agglomerates. As for the zinc oxide nanoparticles synthesized in Example 1 (Figure 4B), they form agglomerates of defined petal-shaped sheets 29.83 nm wide and 72.05 nm long. Finally, with regard to the commercial USP zinc oxide nanoparticles (Figure 4C), these are in the form of defined rods of 205 to 500 nm in length and 99 to 177 nm in base, and agglomerates of several shapes.

**Example 7: - Preparation of agrochemical formulations**

Different formulations (formulations A to H) were tested according to the concentrations shown in Tables 1 to 5. For this purpose, two initial compositions were made. The first one was prepared by initially mixing an aqueous solution of $K_2SO_3$ 40% w/v with soybean oil, then Tween 80 and Cirrasol were added and finally Xanthan gum was added. The second composition was prepared by initially mixing the ZnO nanoparticles with ammonium citrate and glycerol in a ball mill, then mixing by ultrasound for 10 minutes. Finally, both compositions were mixed. As shown in Tables 1 to 5, not all formulations comprise all components. In case one of them is absent, the foregoing procedure was simply continued by skipping the step related to such component. Alternatively, after preparing a concentrated formulation according to the foregoing method, it was diluted to achieve other concentrations. For example, the formulations of Tables 2 to 5 were also prepared by dilution in water from the concentrated formulation of Table 1. Accordingly, the formulation of Table 2 shows a dilution factor equal to 40 with regard to the formulation of Table 1, the formulation of Table 3 shows a dilution factor equal to 200 with regard to the formulation of Table 1, the formulation of Table 4 shows a dilution factor equal to 400 with regard to the formulation of Table 1 and the formulation of Table 5 shows a dilution factor equal to 800 with regard to the formulation of Table 1.

**Table 1.** Ingredients and Concentrations of Concentrated Formulations A through H (NA: Not Applicable)

| Formulation | Potassium silicate (g/L) | Soy oil (%v/v) | Tween 80 (& v/v) | Cirrasol (%v/v) | Xanthan gum (g/L) | Zinc oxide NPs (g/L) | Glycerol (%v/v) | Ammonium citrate (g/L) | Sodium Hexameta phosphate (g/L) |
|---|---|---|---|---|---|---|---|---|---|
| A | 172 | 3.4 | 0.15 | 0.35 | NA | NA | NA | NA | NA |
| B | NA | 3.4 | 0.15 | 0.35 | NA | NA | NA | NA | NA |
| C | NA | NA | NA | NA | 200 | NA | NA | NA | 40 |
| D | NA | NA | NA | NA | 200 | 25.5 | 25.5 | 200 | NA |
| E | 20.416 | NA | NA | NA | 200 | NA | NA | NA | 40 |
| F | 20.416 | NA | NA | NA | 200 | 25.5 | 25.5 | 200 | NA |
| G | 20.416 | 3.4 | 0.15 | 0.35 | 200 | NA | NA | NA | 40 |
| H | 20.416 | 3.4 | 0.15 | 0.35 | 200 | 25.5 | 25.5 | 200 | NA |

**Table 2.** Ingredients and concentrations of formulations A to H with dilution factor 40 with regard to the concentrations in Table 1 (NA: Not applicable).

| Formulation | Potassium silicate (g/L) | Soy oil (%v/v) | Tween 80 (& v/v) | Cirrasol (%v/v) | Xanthan gum (g/L) | Zinc oxide NPs (g/L) | Glycerol (%v/v) | Ammonium citrate (g/L) | Sodium Hexameta phosphate (g/L) |
|---|---|---|---|---|---|---|---|---|---|
| A | 4.3 | 0.085 | 0.00375 | 0.00875 | 0.005 | NA | NA | NA | NA |
| B | NA | 0.085 | 0.00375 | 0.00875 | 0.005 | NA | NA | NA | NA |
| C | NA | NA | NA | NA | 1.53125 | 5 | NA | NA | 1 |
| D | NA | NA | NA | NA | 1.53125 | 5 | 0.6375 | 5 | NA |
| E | 0.5104 | NA | NA | NA | 1.53125 | 5 | NA | NA | 1 |
| F | 0.5104 | NA | NA | NA | 1.53125 | 5 | 0.6375 | 5 | NA |
| G | 0.5104 | 0.085 | 0.00375 | 0.00875 | 1.53125 | 5 | NA | NA | 1 |
| H | 0.5104 | 0.085 | 0.00375 | 0.00875 | 1.53125 | 5 | 0.6375 | 5 | NA |

Table 3. Ingredients and concentrations of formulations A to H with dilution factor 200 with regard to the concentrations in Table 1 (NA: Not applicable).

| Formulation | Potassium silicate (g/L) | Soy oil (%v/v) | Tween 80 (& v/v) | Cirrasol (%v/v) | Xanthan gum (g/L) | Zinc oxide NPs (g/L) | Glycerol (%v/v) | Ammonium citrate (g/L) | Sodium Hexameta phosphate (g/L) |
|---|---|---|---|---|---|---|---|---|---|
| A | 0.86 | 0.017 | 0.00075 | 0.00175 | 0.001 | NA | NA | NA | NA |
| B | NA | 0.017 | 0.00075 | 0.00175 | 0.001 | NA | NA | NA | NA |
| C | NA | NA | NA | NA | 0.30625 | 1 | NA | NA | 0.2 |
| D | NA | NA | NA | NA | 0.30625 | 1 | 0.1275 | 1 | NA |
| E | 0.10208 | NA | NA | NA | 0.30625 | 1 | NA | NA | 0.2 |
| F | 0.10208 | NA | NA | NA | 0.30625 | 1 | 0.1275 | 1 | NA |
| G | 0.10208 | 0.017 | 0.00075 | 0.00175 | 0.30625 | 1 | NA | NA | 0.2 |
| H | 0.10208 | 0.017 | 0.00075 | 0.00175 | 0.30625 | 1 | 0.1275 | 1 | NA |

**Table 4.** Ingredients and concentrations of formulations A to H with dilution factor 400 with regard to the concentrations in Table 1 (NA: Not applicable).

| Formulation | Potassium silicate (g/L) | Soy oil (%v/v) | Tween 80 (& v/v) | Cirrasol (%v/v) | Xanthan gum (g/L) | Zinc oxide NPs (g/L) | Glycerol (%v/v) | Ammonium citrate (g/L) | Sodium Hexameta phosphate (g/L) |
|---|---|---|---|---|---|---|---|---|---|
| A | 0.43 | 0.0085 | 0.000375 | 0.000875 | 0.0005 | NA | NA | NA | NA |
| B | NA | 0.0085 | 0.000375 | NA | 0.0005 | NA | NA | NA | NA |
| C | NA | NA | NA | NA | 0.153125 | 0.5 | NA | NA | 0.1 |
| D | NA | NA | NA | NA | 0.153125 | 0.5 | 0.06375 | 0.5 | NA |
| E | 0.05104 | NA | NA | NA | 0.153125 | 0.5 | NA | NA | 0.1 |
| F | 0.05104 | NA | NA | NA | 0.153125 | 0.5 | 0.06375 | 0.5 | NA |
| G | 0.05104 | 0.0085 | 0.000375 | 0.000875 | 0.153125 | 0.5 | NA | NA | 0.1 |
| H | 0.05104 | 0.0085 | 0.000375 | 0.000875 | 0.153125 | 0.5 | 0.06375 | 0.5 | NA |

**Table 5.** Ingredients and concentrations of formulations A to H with dilution factor 800 with regard to the concentrations in Table 1 (NA: Not applicable).

| Formulation | Potassium silicate (g/L) | Soy oil (%v/v) | Tween 80 (& v/v) | Cirrasol (%v/v) | Xanthan gum (g/L) | Zinc oxide NPs (g/L) | Glycerol (%v/v) | Ammonium citrate (g/L) | Sodium Hexameta phosphate (g/L) |
|---|---|---|---|---|---|---|---|---|---|
| A | 0.215 | 0.00425 | 0.0001875 | 0.0004375 | 0.00025 | NA | NA | NA | NA |
| B | NA | 0.00425 | 0.0001875 | 0.0004375 | 0.00025 | NA | NA | NA | NA |
| C | NA | NA7 | NA | NA | 0.0765625 | 0.25 | NA | NA | 0.05 |
| D | NA | NA | NA | NA | 0.0765625 | 0.25 | 0.375 | 0.25 | NA |
| E | 0.02552 | NA | NA | NA | 0.0765625 | 0.25 | NA | NA | 0.05 |
| F | 0.02552 | NA | NA | NA | 0.0765625 | 0.25 | 0.375 | 0.25 | NA |
| G | 0.02552 | 0.0001875 | 0.0001875 | 0.0004375 | 0.0765625 | 0.25 | NA | NA | 0.05 |
| H | 0.02552 | 0.0001875 | 0.0001875 | 0.0004375 | 0.0765625 | 0.25 | 0.375 | 0.25 | NA |

**Example 8: - Stability evaluation of the invention formulations**

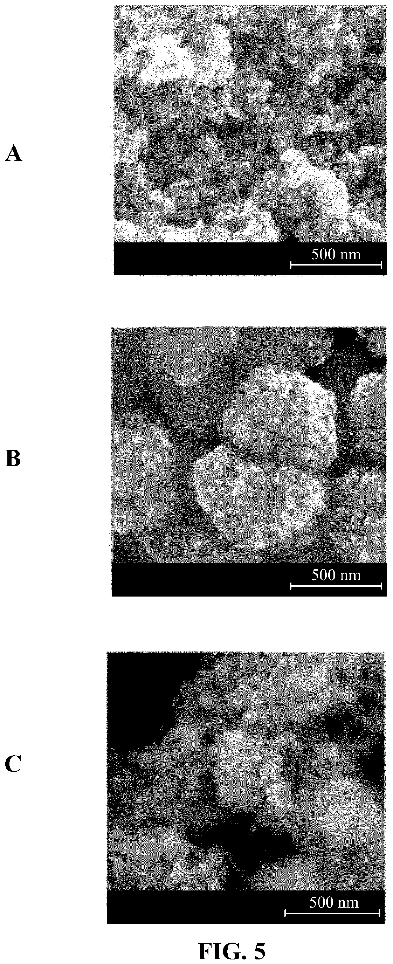

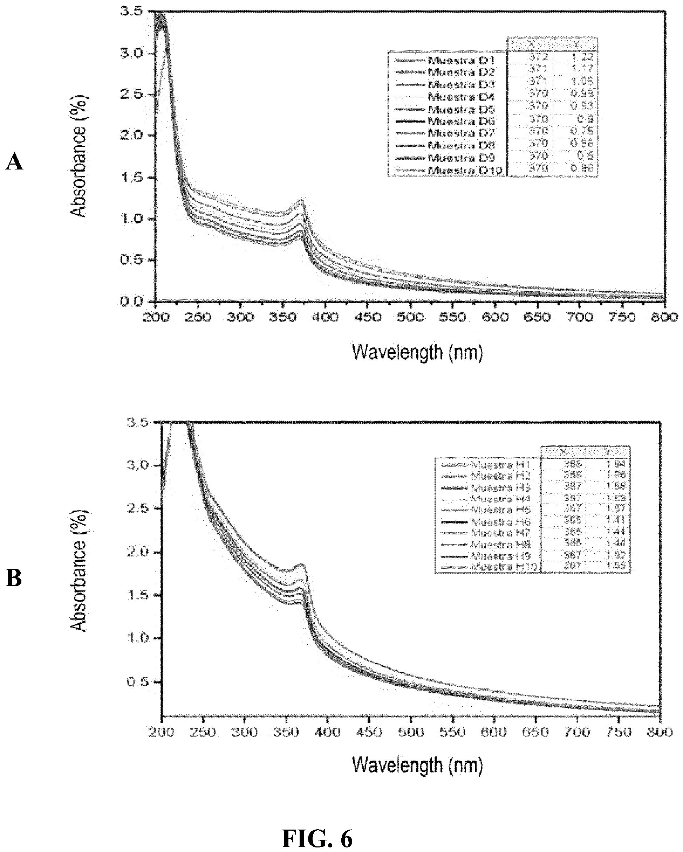

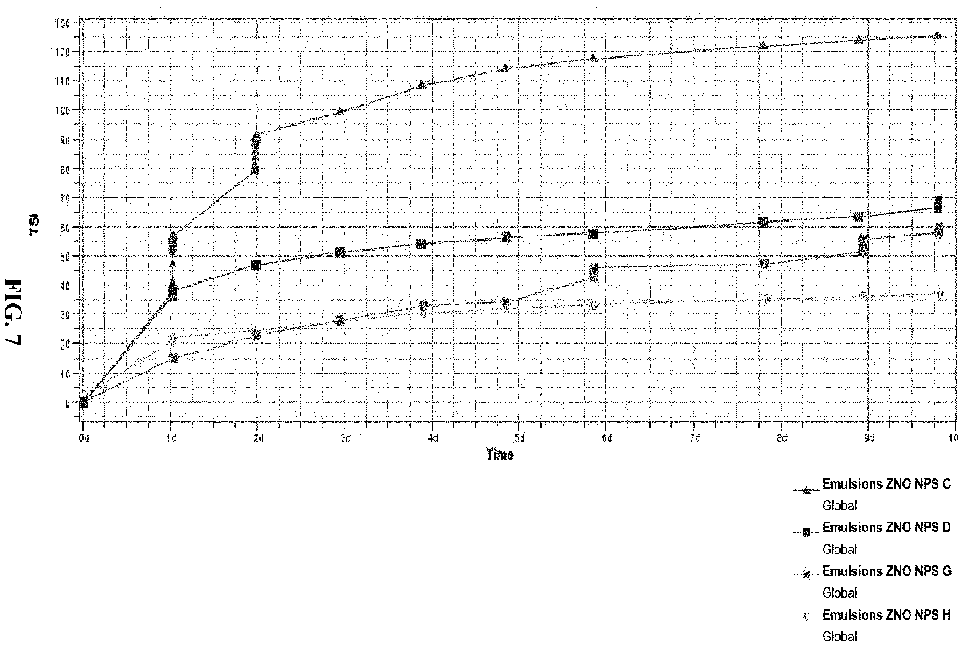

The stability of the formulations D, F and H was determined through EDS, SEM (FIG. 5), UV-Vis spectrometry (FIG. 6A and 6B) and Turbiscan techniques, the latter two to evaluate the stability of the formulations in a 10-day period of time. Considering the EDS results, the presence of zinc, oxygen and carbon in formulation D is evident. In addition, this formulation presents zinc oxide nanoparticles with particle size between 42 to 60 nm, spherical shapes, with abundant agglomeration of irregular shapes and totally coated (FIG. 5A). As for formulation F, it is characterized by the presence of zinc, oxygen, carbon, silicon and potassium. In addition, this formulation presents zinc oxide nanoparticles with a particle size between 22 to 47.4 nm, spherical shapes, with spherical agglomeration with diameter sizes from 542.0 nm to 638.2 nm, within a linear network covering them, given by the emulsifying compounds of the formulation (FIG. 5B). Finally, in formulation H the presence of zinc, oxygen, silicon and potassium is evidenced. The zinc oxide nanoparticles of formulation H have a particle size between 24.0 to 68.0 nm, spherical shapes with varied agglomeration, spherical with spherical deformations and polygons, within linear networks that surround and cover them, given by the emulsifying compounds of the formulation (FIG. 5C). All the formulations evaluated show high stability over long periods of time as evidenced in FIG. 7.

**Example 9: - Antifungal activity evaluation of the formulations in Example 7**

The antifungal activity of formulations D, F, H, I and J of Example 7 and ½ and ¼ dilutions thereof were tested on four microorganisms: *Botrytis cinerea* ATCC 36634, *Mycosphaerella fijiensis* ATCC 36055, *Phytophthora palmivora* ATCC 46634 and *Phytophthora infestans* ATCC 48716. In order to compare the effect of the formulations of the present invention, commercial fungicides and oomyceticides were also tested: Misilk 360, Nitrofil FT, Siganex, Mancozeb 80% WP and Forum 500 WP. Additionally, in order to determine the effect of the size of the ZnO nanoparticles, the foregoing formulations were prepared using commercial zinc oxide USP, characterized by a particle size larger than the nanoparticles used in the formulations of the invention. The formulations containing commercial USP zinc oxide are designated with an asterisk (*).

The fungi and oomycetes were grown on 2% potato dextrose agar (PDA) at a temperature of 24 ± 1°C for 7 to 30 days of incubation, depending on the strain. Subsequently, new PDA medium containing the formulations of the invention was inoculated. The formulations were used at the concentration shown in Tables 3, 4 and 5 and sterile distilled water was used as a control. Then, using a Vernier caliper, the radial growth of the pathogen was measured based on time. All assays were performed three times. The formula used to calculate the percentage of inhibition is as follows:

$$Inhibition\ Percentage = \frac{Control\ diameter - Treatment\ diameter}{Control\ diameter\ x\ 100}$$

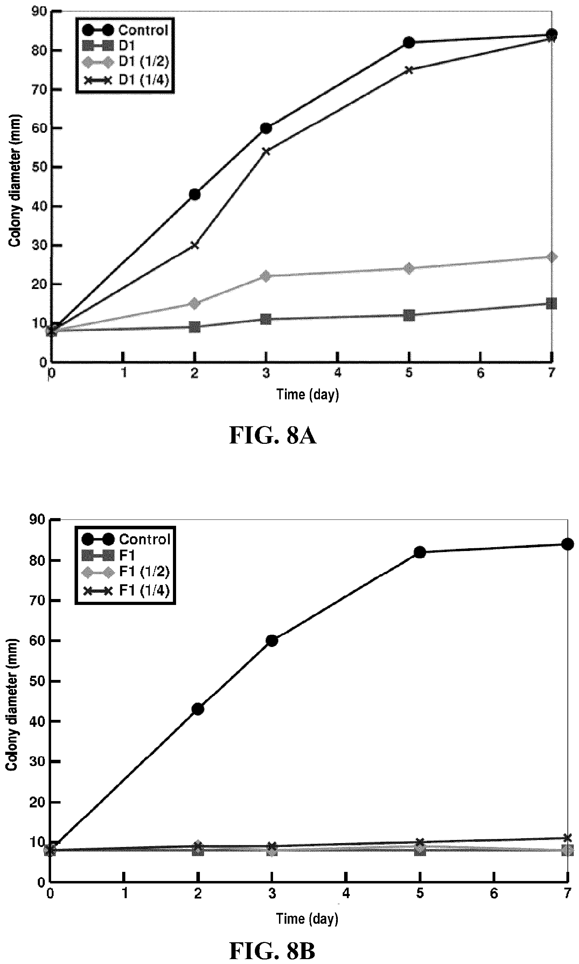

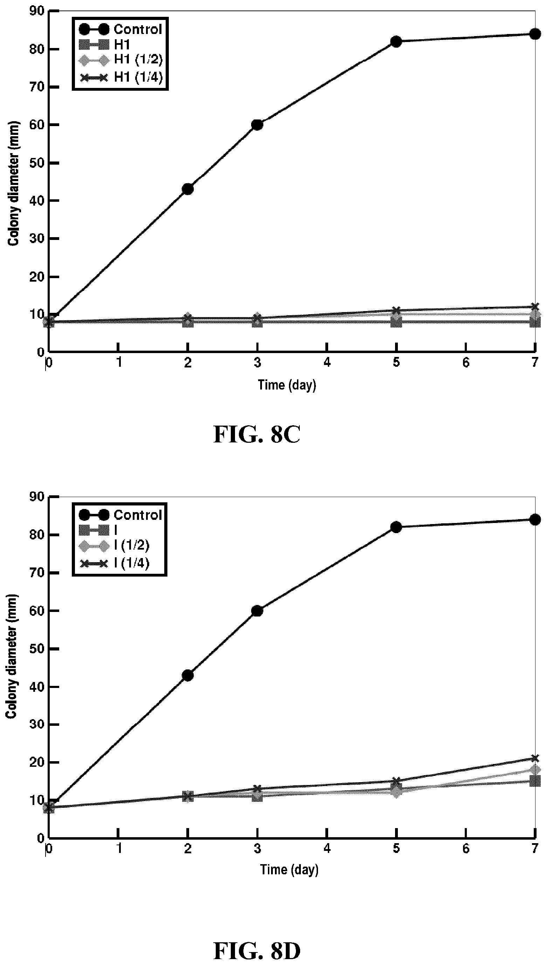

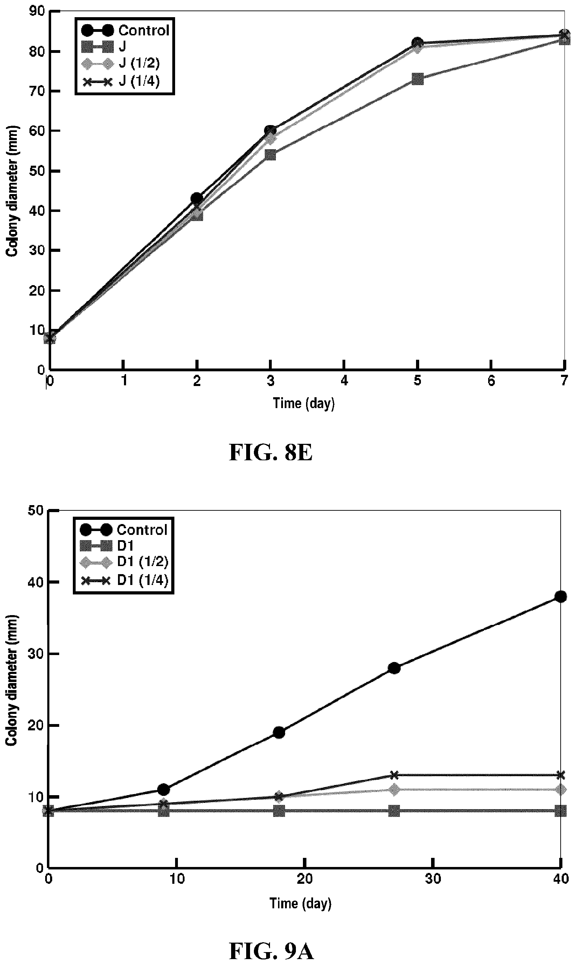

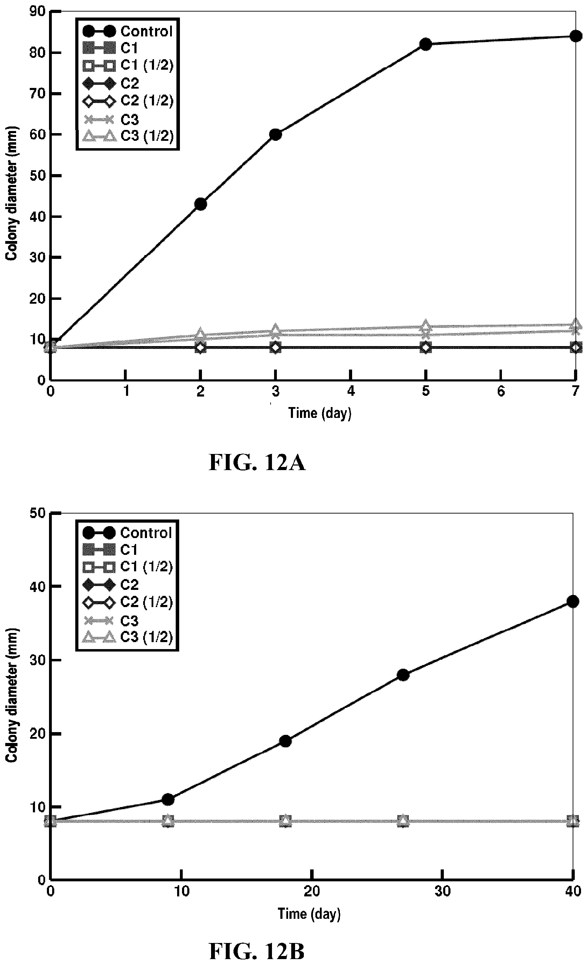

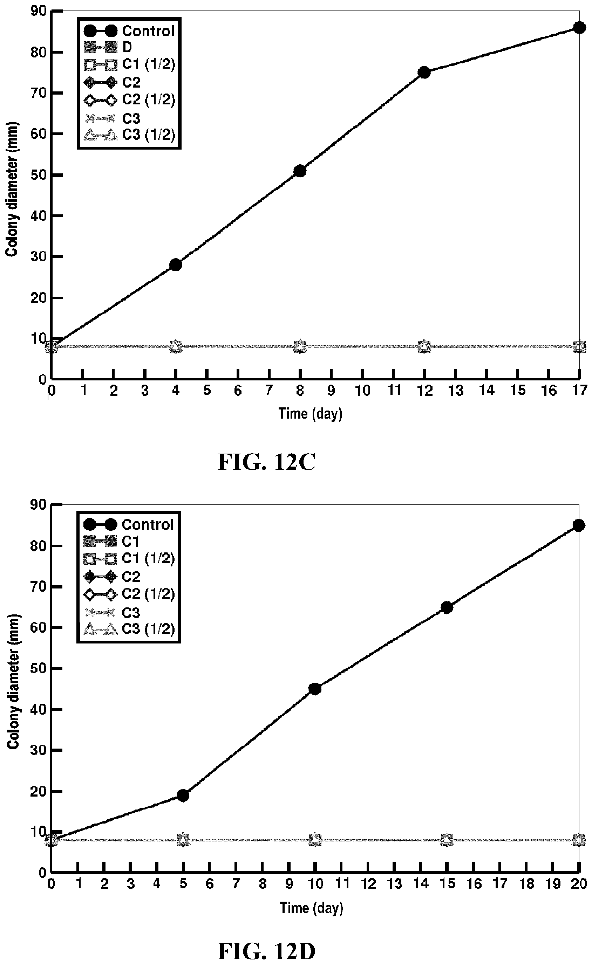

Table 6 presents the results obtained for *B. cinerea* ATCC 36634 after 7 days of growth for the formulations evaluated (FIG. 8A, 8B, 8C, 8D and 8E). According to the results obtained at day 7 of the assay, formulations F and H were those that exhibited the highest *in vitro* antifungal activity on the *Botrytis cinerea* strain with growth inhibition percentages between 80 to 99% with regard to the growth control without antifungals. The antifungal activity of the highest evaluated concentration of formulations F and H was comparable to that exhibited by the commercial fungicides Siganex and Mancozeb (FIG. 12A).

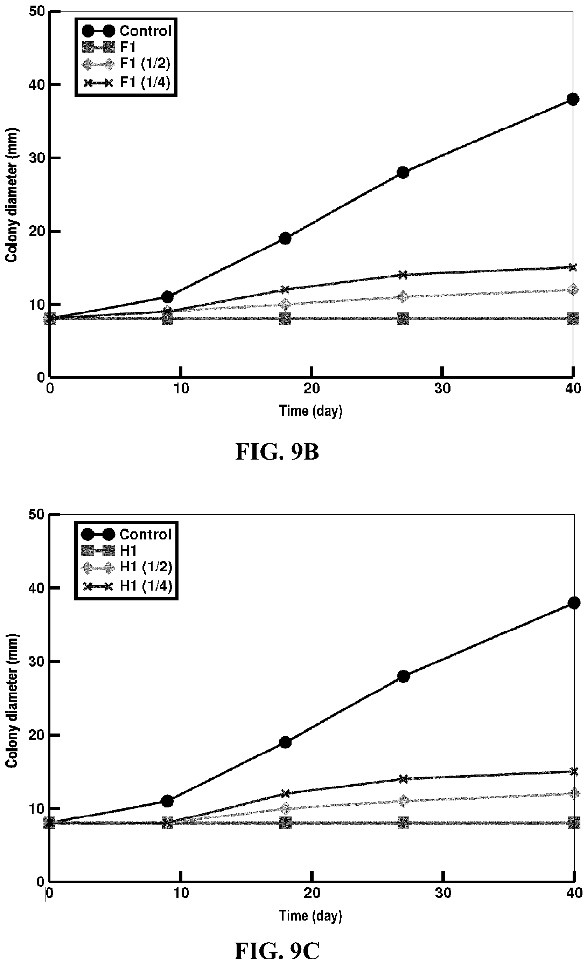

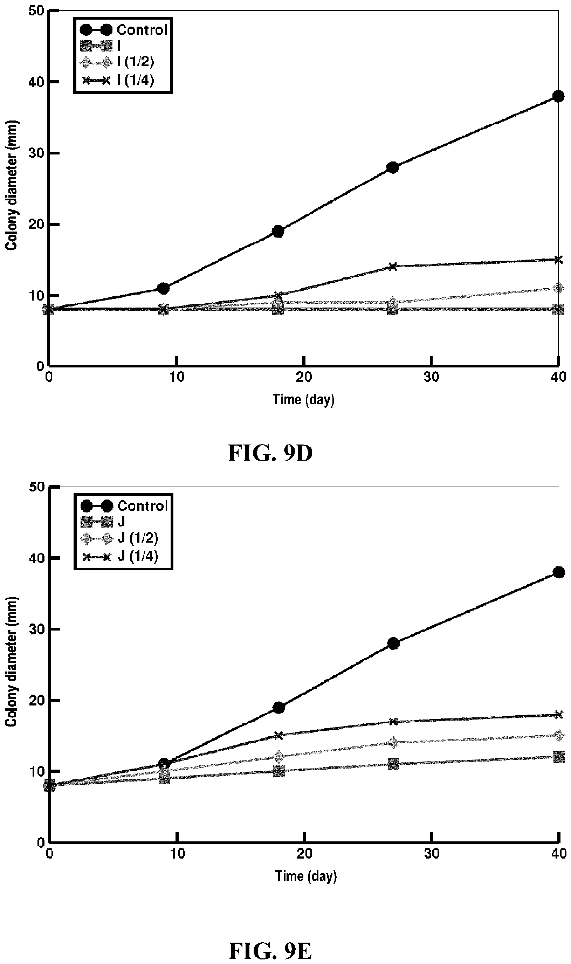

The results of growth inhibition of *M. fijiensis* ATCC 36055 for the evaluated formulations (FIG. 9A, 9B, 9C, 9D and 9E) at day 40 are shown in Table 7. The growth of this fungus was stabilized even up to day 55. For this reason, day 40 was considered as the maximum point of fungus radial growth. Considering the results, formulations D, H and H* exhibited the highest *in vitro* antifungal activity on *M. fijiensis* strain, inhibiting 100% of the fungal growth, as well as the commercial fungicides evaluated as inhibition control (FIG. 12B).

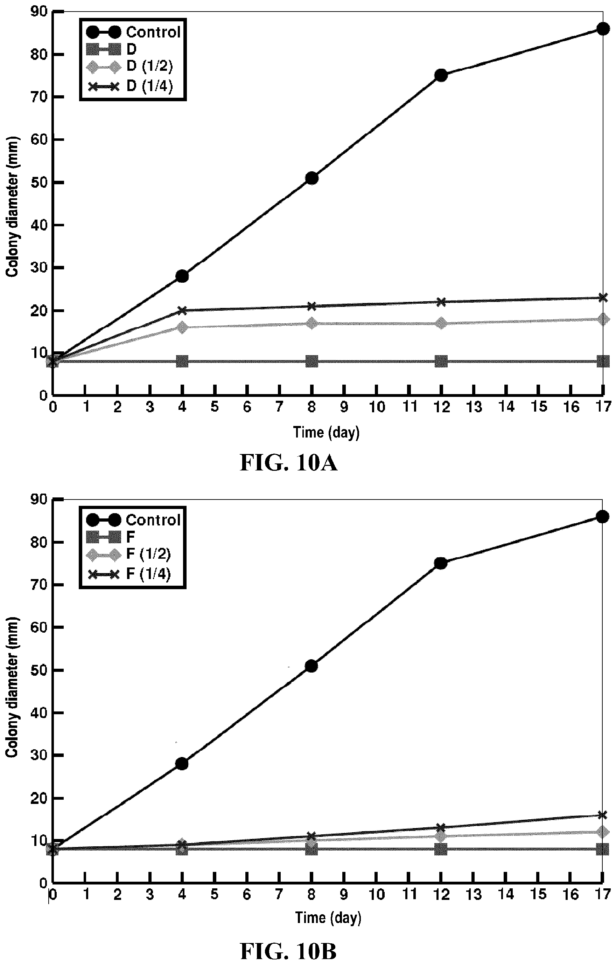

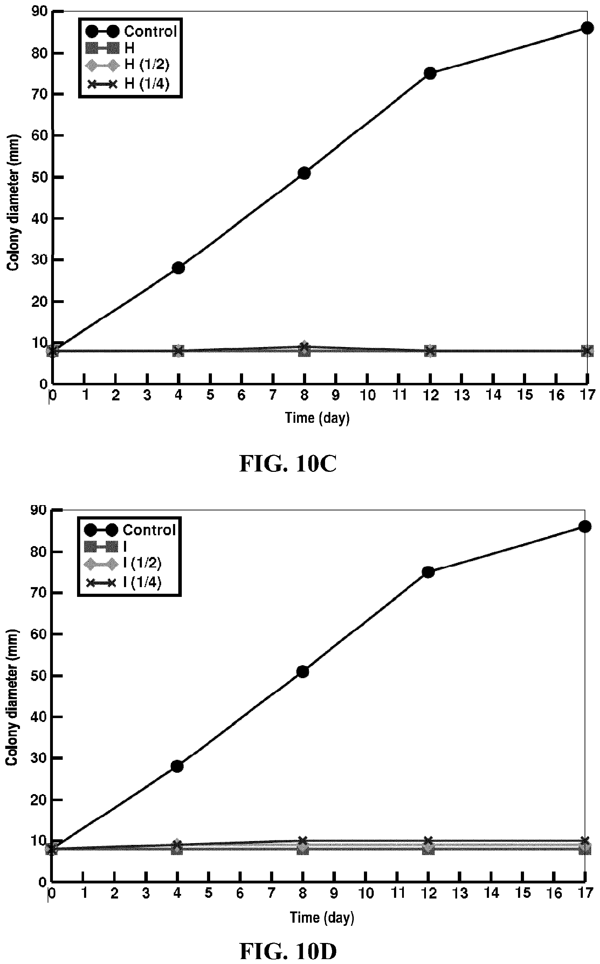

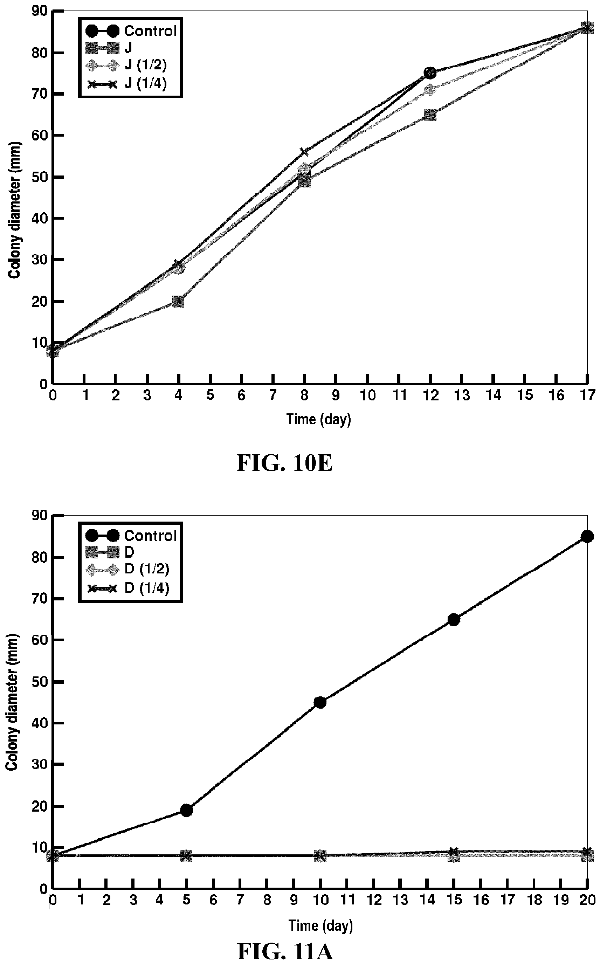

Similarly, Table 8 presents the results obtained for the strain *P. palmivora* ATCC 46634 for the formulations evaluated (FIG. 10A, 10B, 10C, 10D and 10E) at day 17 of the study. According to the results, compounds F, H, H* and Misilk 360 (FIG. 12C) exhibited the highest *P. palmivora* activity even at the lowest concentrations evaluated (dilution factor 800), exhibiting growth inhibition percentages higher than 94% with regard to the control. The maximum concentration of the evaluated formulations inhibited more than 99% of the oomycete, with the exception of the compound Nitrofil FT.

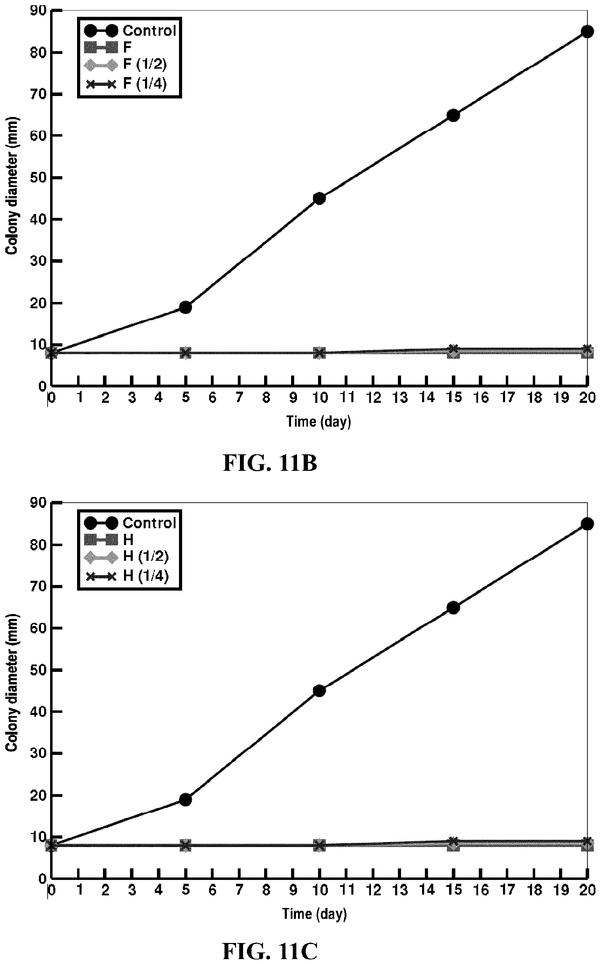

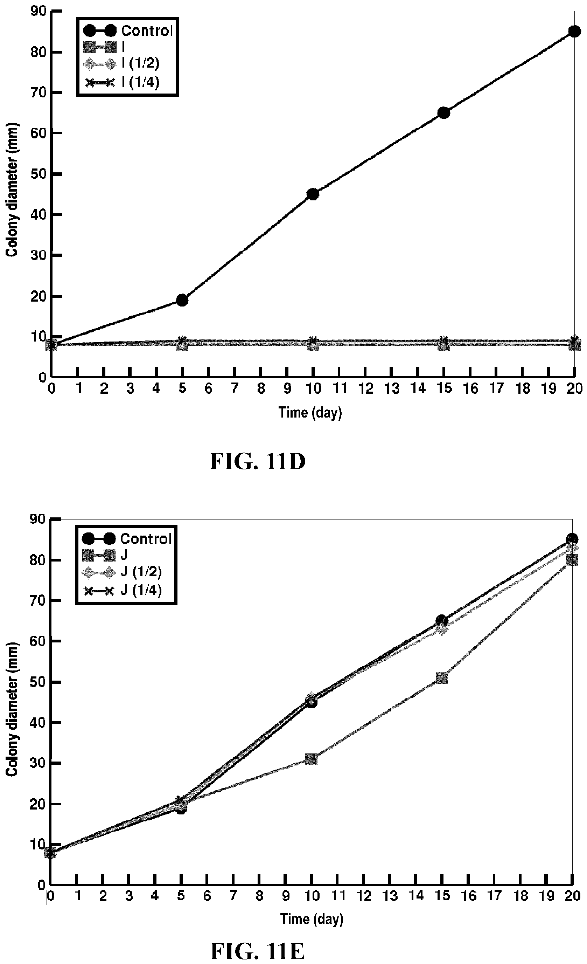

The results of growth inhibition of *P. infestans* ATCC 46634 for the evaluated formulations (FIG. 11A, 11B, 11C, 11D and 11E) at day 20 are shown in Table 9. According to the results obtained, with the exception of compound (J), the formulations of the invention may inhibit the growth *of P. infestans* by 98 to 100%.

In conclusion, it may be considered that the highest concentration of the formulations F, F*, H and H* are the best formulations with antifungal or antioomycete activity *in vitro* on the different phytopathogens evaluated, being comparable to the commercial fungicides Siganex, Mancozeb 80%WP and Forum 500 WP.

**Table** 6. Growth inhibition of *B. cinerea* ATCC 36634 against the invention formulations. The formulations marked with asterisk (*) were prepared using commercial zinc oxide nanoparticles (USP).

| Formulation | Dilution Factor: 200 | | Dilution Factor: 400 | | Dilution Factor: 800 | |
|---|---|---|---|---|---|---|
| | Colony Diameter (mm) ± Standard Deviation | Growth Inhibition Percentage (%) | Colony Diameter (mm) ± Standard Deviation | Growth Inhibition Percentage (%) | Colony Diameter (mm) ± Standard Deviation | Growth Inhibition Percentage (%) |
| White | 86.00 ± 0.00 | - | 86.00 ± 0.00 | - | 86.00 ± 0.00 | - |
| A | - | - | - | - | - | - |
| B | - | - | - | - | - | - |
| C | - | - | - | - | - | - |
| C* | - | - | - | - | - | - |
| D | 19.14 ± 0.31 | 85.39 | 30.17 ± 0.59 | 71.30 | 85.20 ± 0.72 | 1.02 |
| D* | 27.69 ± 0.90 | 74.47 | 63.67 ± 0.77 | 28.52 | 85.00 ± 1.00 | 1.28 |
| E | - | - | - | - | - | - |
| E* | - | - | - | - | - | - |
| F | 8.33 ± 0.42 | 99.20 | 8.60 ± 0.36 | 98.85 | 10.07 ± 0.31 | 96.97 |
| F* | 11.57 ± 0.51 | 95.06 | 18.53 ± 0.50 | 86.17 | 22.23 ± 0.40 | 81.44 |
| G | - | - | - | - | - | - |
| G* | - | - | - | - | - | - |
| H | 9.37 ± 0.32 | 97.87 | 12.23 ± 0.64 | 94.21 | 15.27 ± 0.64 | 90.33 |
| H* | 9.77 ± 0.68 | 97.36 | 12.60 ± 0.40 | 97.74 | 23.07 ± 1.10 | 80.37 |
| Misilk 360 | 17.63 ± 0.32 | 87.32 | 19.93 ± 1.01 | 84.38 | 23.13 ± 0.81 | 80.29 |
| Nitrofil FT | 85.80 ± 0.35 | 0.26 | 85.67 ± 0.58 | 0.42 | 86.00 ± 0.00 | 0.00 |
| Siganex | 7.70 ± 0.00 | 100.00 | 7.70 ± 0.00 | 100.00 | | - |
| Mancozeb 80% WP | 7.70 ± 0.00 | 100.00 | 7.70 ± 0.00 | 100.00 | - | - |
| Forum 500 WP | 11.07 ± 0.30 | 95.70 | 11.90 ± 0.20 | 94.64 | - | - |

**Table 7.** Growth inhibition of *M. fijiensis* ATCC 36055 against the invention formulations. The formulations marked with asterisk (*) were prepared using commercial (USP) zinc oxide nanoparticles.

| Formulation | Dilution Factor: 200 | | Dilution Factor: 400 | | Dilution Factor: 800 | |
|---|---|---|---|---|---|---|
| | Colony Diameter (mm) ± Standard Deviation | Growth Inhibition Percentage (%) | Colony Diameter (mm) ± Standard Deviation | Growth Inhibition Percentage (%) | Colony Diameter (mm) ± Standard Deviation | Growth Inhibition Percentage (%) |
| White | 38.07 ± 0.00 | - | 38.07 ± 0.00 | - | 38.07 ± 0.00 | - |
| A | - | - | - | - | - | - |
| B | - | - | - | - | - | - |
| C | - | - | - | - | - | - |

(continued)

| Formulation | Dilution Factor: 200 | | Dilution Factor: 400 | | Dilution Factor: 800 | |
| --- | --- | --- | --- | --- | --- | --- |
| | Colony Diameter (mm) ± Standard Deviation | Growth Inhibition Percentage (%) | Colony Diameter (mm) ± Standard Deviation | Growth Inhibition Percentage (%) | Colony Diameter (mm) ± Standard Deviation | Growth Inhibition Percentage (%) |
| C* | - | - | - | - | - | - |
| D | 7.70 ± 0.00 | 100.00 | 9.47 ± 0.31 | 94.18 | 10.73 ± 0.25 | 90.01 |
| D* | 10.07 ± 0.50 | 92.21 | 12.43 ± 0.71 | 84.41 | 14.27 ± 0.64 | 78.38 |
| E | - | - | - | - | - | - |
| E* | - | - | - | - | - | - |
| F | 7.80 ± 0.17 | 99.67 | 13.17 ± 0.72 | 82.00 | 16.43 ± 0.78 | 71.24 |
| F* | 8.23 ± 0.06 | 98.24 | 10.23 ± 0.25 | 91.66 | 11.40 ± 0.36 | 87.82 |
| G | - | - | - | - | - | - |
| G* | - | - | - | - | - | - |
| H | 7.70 ± 0.00 | 100.00 | 10.80 ± 0.72 | 89.79 | 14.93 ± 0.61 | 76.18 |
| H* | 7.70 ± 0.00 | 100.00 | 12.30 ± 0.46 | 84.85 | 15.83 ± 0.76 | 73.22 |
| Misilk 360 | 8.53 ± 0.42 | 97.26 | 9.17 ± 0.29 | 95.17 | 10.70 ± 0.44 | 90.12 |
| Nitrofil FT | 11.77 ± 0.21 | 86.61 | 14.63 ± 0.60 | 77.17 | 17.83 ± 0.35 | 66.63 |
| Siganex | 7.70 ± 0.00 | 100.00 | 7.70 ± 0.00 | 100.00 | - | - |
| Mancozeb 80% WP | 7.70 ± 0.00 | 100.00 | 7.70 ± 0.00 | 100.00 | - | - |
| Forum 500 WP | 7.70 ± 0.00 | 100.00 | 7.70 ± 0.00 | 100.00 | - | - |

**Table 8.** Growth inhibition of *P. palmivora* ATCC 46634 against the invention formulations. Formulations marked with an asterisk (*) were prepared using commercial zinc oxide nanoparticles (USP).

| Formulation | Dilution Factor: 200 | | Dilution Factor: 400 | | Dilution Factor: 800 | |
| --- | --- | --- | --- | --- | --- | --- |
| | Colony Diameter (mm) ± Standard Deviation | Growth Inhibition Percentage (%) | Colony Diameter (mm) ± Standard Deviation | Growth Inhibition Percentage (%) | Colony Diameter (mm) ± Standard Deviation | Growth Inhibition Percentage (%) |
| White | 86.00 ± 0.00 | - | 86.00 ± 0.00 | - | 86.00 ± 0.00 | - |
| A | - | - | - | - | - | - |
| B | - | - | - | - | - | - |
| C | - | - | - | - | - | - |
| C* | - | - | - | - | - | - |
| D | 8.33 ± 0.15 | 99.19 | 18.37 ± 0.78 | 86.38 | 23.07 ± 0.31 | 80.37 |
| D* | 8.10 ± 0.17 | 99.49 | 62.67 ± 1.60 | 29.80 | 79.00 ± 1.40 | 8.94 |
| E | - | - | - | - | - | - |
| E* | - | - | - | - | - | - |

(continued)

| Formulation | Dilution Factor: 200 | | Dilution Factor: 400 | | Dilution Factor: 800 | |
|---|---|---|---|---|---|---|
| | Colony Diameter (mm) ± Standard Deviation | Growth Inhibition Percentage (%) | Colony Diameter (mm) ± Standard Deviation | Growth Inhibition Percentage (%) | Colony Diameter (mm) ± Standard Deviation | Growth Inhibition Percentage (%) |
| F | 8.13 ± 0.15 | 99.45 | 9.60 ± 0.53 | 97.57 | 11.80 ± 0.72 | 94.76 |
| F* | 8.33 ± 0.21 | 99.19 | 9.93 ± 0.12 | 97.15 | 20.53 ± 1.86 | 83.61 |
| G | - | - | - | - | - | - |
| G* | - | - | - | - | - | - |
| H | 8.00 ± 0.00 | 99.62 | 8.00 ± 0.00 | 99.62 | 8.00 ± 0.00 | 99.62 |
| H* | 8.00 ± 0.00 | 99.62 | 8.30 ± 0.26 | 99.23 | 11.27 ± 0.64 | 95.44 |
| Misilk 360 | 8.23 ± 0.21 | 99.32 | 8.63 ± 0.15 | 98.81 | 8.60 ± 0.30 | 98.85 |
| Nitrofil FT | 86.00 ± 0.00 | 0.00 | 86.00 ± 0.00 | 0.00 | 86.00 ± 0.00 | 0.00 |
| Siganex | 7.70 ± 0.00 | 100.00 | 7.70 ± 0.00 | 100.00 | - | - |
| Mancozeb 80% WP | 7.70 ± 0.00 | 100.00 | 7.70 ± 0.00 | 100.00 | - | - |
| Forum 500 WP | 7.70 ± 0.00 | 100.00 | 7.70 ± 0.00 | 100.00 | - | - |

**Table 9.** Growth inhibition of *P. infestans* ATCC 48716 against the invention formulations. The formulation marked with asterisk (*) were prepared using commercial zinc oxide nanoparticles (USP).

| Formulation | Dilution Factor: 200 | | Dilution Factor: 400 | | Dilution Factor: 800 | |
|---|---|---|---|---|---|---|
| | Colony Diameter (mm) ± Standard Deviation | Growth Inhibition Percentage (%) | Colony Diameter (mm) ± Standard Deviation | Growth Inhibition Percentage (%) | Colony Diameter (mm) ± Standard Deviation | Growth Inhibition Percentage (%) |
| White | 86.00 ± 0.00 | - | 86.00 ± 0.00 | - | 86.00 ± 0.00 | - |
| A | - | - | - | - | - | - |
| B | - | - | - | - | - | - |
| C | - | - | - | - | - | - |
| C* | - | - | - | - | - | - |
| D | 7.70 ± 0.00 | 100.00 | 7.70 ± 0.00 | 100.00 | 9.53 ± 0.50 | 97.66 |
| D* | 7.70 ± 0.00 | 100.00 | 8.23 ± 0.25 | 99.32 | 17.53 ± 0.50 | 87.44 |
| E | - | - | - | - | - | - |
| E* | - | - | - | - | - | - |
| F | 7.70 ± 0.00 | 100.00 | 8.17 ± 0.15 | 99.40 | 8.90 ± 0.36 | 98.47 |
| F* | 7.70 ± 0.00 | 100.00 | 7.70 ± 0.00 | 100.00 | 7.70 ± 0.00 | 100.00 |
| G | - | - | - | - | - | - |
| G* | - | - | - | - | - | - |
| H | 8.70 ± 0.36 | 98.72 | 9.10 ± 0.26 | 98.21 | 9.37 ± 0.15 | 97.87 |

(continued)

| Formulation | Dilution Factor: 200 | | Dilution Factor: 400 | | Dilution Factor: 800 | |
|---|---|---|---|---|---|---|
| | Colony Diameter (mm) ± Standard Deviation | Growth Inhibition Percentage (%) | Colony Diameter (mm) ± Standard Deviation | Growth Inhibition Percentage (%) | Colony Diameter (mm) ± Standard Deviation | Growth Inhibition Percentage (%) |
| H* | 7.70 ± 0.00 | 100.00 | 7.70 ± 0.00 | 100.00 | 7.70 ± 0.00 | 100.00 |
| Misilk 360 | 7.70 ± 0.00 | 100.00 | 7.70 ± 0.00 | 100.00 | 8.57 ± 0.40 | 98.89 |
| Nitrofil FT | 80.33 ± 1.53 | 7.24 | 83.33 ± 1.53 | 3.41 | 85.67 ± 0.58 | 0.43 |
| Siganex | 7.70 ± 0.00 | 100.00 | 7.70 ± 0.00 | 100.00 | - | - |
| Mancozeb 80% WP | 7.70 ± 0.00 | 100.00 | 7.70 ± 0.00 | 100.00 | - | - |
| Forum 500 WP | 7.70 ± 0.00 | 100.00 | 7.70 ± 0.00 | 100.00 | - | - |

**Claims**

1. An agrochemical formulation comprising:

   - a silicate salt;
   - a vegetable oil;
   - a surfactant or a mixture of surfactants;
   - a dispersant or a mixture of dispersants;
   - zinc oxide nanoparticles; and
   - a stabilizer or mixture of stabilizers.

2. The agrochemical formulation according to Claim 1, wherein the stabilizer or mixture of stabilizers is a thickener.

3. The agrochemical formulation according to Claim 1, wherein the formulation is in the form of an emulsion.

4. The agrochemical formulation according to Claim 1, wherein the silicate salt is selected from the group consisting of sodium silicate, potassium silicate, manganese silicate, magnesium silicate or calcium silicate.

5. The agrochemical formulation of Claim 1, wherein the silicate salt is in a concentration between 0.01 and 250 g/L.

6. The agrochemical formulation according to Claim 1, wherein the surfactant is selected from the group consisting of ethoxylated linear alcohols, ethoxylated alkylphenols, fatty acid esters, amine derivatives, amide derivatives, alkylpolyglucosides, ethylene glycol/propylene glycol copolymers, polyols, ethoxylated polyols, thiols (mercaptans), or a mixture thereof.

7. The agrochemical formulation of Claim 1, wherein the surfactant is in a concentration between 0.01 and 10 g/L.

8. The agrochemical formulation according to Claim 1, wherein the vegetable oil is selected from the group consisting of soybean oil, corn oil, cottonseed oil, olive oil, palm oil, peanut oil, canola oil, safflower oil, sesame oil, sunflower oil, hazelnut oil, almond oil, walnut oil, macadamia oil, pecan oil, and pistachio oil.

9. The agrochemical formulation of Claim 1, wherein the vegetable oil is in a concentration between 0.01 and 50 g/L.

10. The agrochemical formulation of Claim 1, wherein the dispersant is selected from the group consisting of glycerol, ammonium citrate or sodium hexametaphosphate.

11. The agrochemical formulation of Claim 1, wherein the dispersant is in a concentration between 0.1 and 500 g/L.

**12.** The agrochemical formulation according to Claim 1, wherein the stabilizer is selected from the group consisting of polyvinyl alcohol, methylcellulose, xanthan gum, hydroxymethylcellulose, gum arabic, carboxymethylcellulose, hydroxyethylcellulose, hydroxypropylcellulose, hydroxypropylmethylcellulose, gellan gum, guar gum, locust bean gum, tragacanth gum, succinoglucan gum, gelatin, carrageenan, starch, sago, tapioca, pectin, collagen and agar.

**13.** The agrochemical formulation of Claim 1, wherein the stabilizer is in a concentration between 0.01 and 5 g/L.

**14.** The agrochemical formulation of Claim 1, wherein the zinc oxide nanoparticles are between 1 and 100 nm in size.

**15.** The agrochemical formulation of Claim 1, wherein the zinc oxide nanoparticles are in a concentration between 0.1 and 300 g/L.

**16.** A method for preparing the agrochemical formulation of Claim 1 comprising the steps of:

> a) mixing an aqueous solution of the silicate salt with a vegetable oil;
> b) adding a surfactant or a mixture of surfactants;
> c) adding a stabilizer or a mixture of stabilizers;
> d) preparing a nanofluid comprising zinc oxide nanoparticles and a dispersant;
> e) mixing the product resulting from step a) with the nanofluid from step d).

**17.** The use of the agrochemical formulation according to any of Claims 1 to 16 for the prevention or control of a plant disease caused by a microorganism.

**18.** The use of Claim 17, wherein the plant is selected from the group consisting of plants producing fruits or infructescences, ornamental plants, medicinal plants, legumes, grains and tubers.

**19.** The use of Claim 18, wherein the plant is selected from the group consisting of chard (*Beta vulgaris),* chili peppers *(Capsicum spp.),* garlic and onions *(Allium spp.* ), celery *(Celery gravolens),* eggplant (*Solanum meolongena*), pumpkin (*Curcurbita moschata*), chayote *(Sechium edule),* cabbage *(Brassica oleracea*), spinach (*Spinaca oleracea*), bean *(Phaseolus vulgaris),* lettuce (*Lactuca sativa*), maize (*Zea mays),* peanut (*Arachis hypogaea*), tomato (*Solanum lycopersicum),* cucumber *(Cucumis sativus),* okra *(Hibiscus esculentus),* radish (*Raphanus sativus),* beetroot (*Beta vulgaris),* carrot (*Daucus carota*), avocado (*Persea auericana*), anon (*Annona squamosa*), caimito (*Chrysophyllum cainito),* canistel (*Pouteria campechiana*), cherry (*Malpighia punicifolia*), custard apple (*Annona reticulata*), plum (*Spondias dulcis*), coconut (*Coco nucifera*), papaya (*Carica papaya*), soursop (*Annona muricata*), guava (*Psidium guajaba*), pomegranate *(Punica granatum),* lime (*Citrus aurantifolium*), lemon *(Citrus limonum),* red mamey *(Calocarpum mammosum),* mamey santo domingo (*Mammea americano*), mamoncillo (*Melicocea bijuga*), mandarin *(Citrus reticulata),* mango *(Mangifera indica),* passion fruit (*Passiflora laurifolia*), water melon *(Citrullus vulgaris),* sour orange *(Citrus aurantium*), sweet orange *(Citrus sinensis),* pineapple (*Ananas comosus*), banana (*Musa paradisiaca),* plantain (*Musa balsisiana*), tamarind (*Tamarindus indica*), grapefruit (*Citrus paradisi),* Creole grapefruit *(Citrus grandin*), grape *(Vitis vinifera),* bean (*Phaseolus vulgaris),* maize (*Zea mays),* rice (*Oryza sativa*), coffee (*Coffea arabica*), sugar cane (*Saccharum officinarum),* cotton (*Gossypium hirsutum),* gherkin (*Melothria guadalupensis*), sweet potato (*Ipomoes batatas*), potato (*Solanum tuberosum),* creole potato (*Solanum phureja*), cape gooseberry (*Physalis peruviana),* cassava (*Manihot esculenta*), soybean *(Glycine max),* strawberry (*Fragaria spp.* ), blackberry *(Morus* spp.), bramble *(Rubus* spp.), palms of the family Aracaceae, oil palm (*Elaeis guineensis*), cocoa (*Theobroma cacao*), tree tomato (*Solanum betaceum),* lulo (*Solanum quitoense).*

**20.** The use according to Claim 16, wherein the microorganism is a fungus or oomycete.

**21.** The use according to Claim 20, wherein the microorganism is a fungus.

**22.** The use according to Claim 21, wherein the fungus is selected from a group comprising *Magnaporthe oryzae, Botrytis cinerea, Puccinia* spp. (including but not limited to *Fusarium graminearum, Fusarium oxysporum), Blumeria graminis, Mycosphaerella spp. Mycosphaerella fijiensis, Mycosphaerella graminicola, Colletotrichum* spp., *Ustilago maydis, Melampsora lini, Phakopsora pachyrhizi* and *Rhizoctonia solani.*

**23.** The use according to Claim 21, wherein the microorganism is an oomycete.

**24.** The use according to Claim 23, wherein the oomycete is selected from a group comprising *Phytophthora* spp, *Phytophthora infestans, Phytophthora ramorum, Phytophthora sojae, Phytophthora capsici, Phytophthora cinnamomi, Phytophthora palmivora and Phytophthora parasitica, Hyaloperonospora arabidopsidis, Plasmopara viticola, Pythium ultimum, Albugo candida, and Peronosporafarinosa.*

**FIG. 1**

**FIG. 2**

**FIG. 3**

A

B

C

**FIG. 4**

A

B

C

**FIG. 5**

**FIG. 6**

**FIG. 7**

FIG. 8A

FIG. 8B

**FIG. 8C**

**FIG. 8D**

FIG. 8E

FIG. 9A

FIG. 9B

FIG. 9C

**FIG. 9D**

**FIG. 9E**

**FIG. 10A**

**FIG. 10B**

**FIG. 10C**

**FIG. 10D**

**FIG. 10E**

**FIG. 11A**

**FIG. 11B**

**FIG. 11C**

FIG. 11D

FIG. 11E

**FIG. 12A**

**FIG. 12B**

**FIG. 12C**

**FIG. 12D**

## INTERNATIONAL SEARCH REPORT

| | International application No. |
|---|---|
| | PCT/IB2020/060115 |

**A. CLASSIFICATION OF SUBJECT MATTER**

A01N 25/22, 25/30, 55/02, 55/10, 59/06, 59/16, A01P 1/00, 3/00 (2021.01)

According to International Patent Classification (IPC) or to both national classification and IPC

**B. FIELDS SEARCHED**

Minimum documentation searched (classification system followed by classification symbols)

(CIP) A01N, A01P

Documentation searched other than minimum documentation to the extent that such documents are included in the fields searched

Electronic data base consulted during the international search (name of data base and, where practicable, search terms used)

Derwent, Patentscope, Esp@cenet, Google Patent, Google, INAPI, BEIC

**C. DOCUMENTS CONSIDERED TO BE RELEVANT**

| Category* | Citation of document, with indication, where appropriate, of the relevant passages | Relevant to claim No. |
|---|---|---|
| Y | WO2014126584A1 (LATITUDE 18, INC.), 21-08-2014, The whole document, especially abstract paragraphs [0001], [0014], [0016], [0023], [0024], [0025], [0027], [0028], [0030]-[0032], [0034]-[0040], [0042], [0044], [0045], [0050]-[0053] and [0068], and claims 1, 3-5, 8, 9, 11, 12-14, 19 and 26. | 1-24 |
| Y | SARDELLA, D. et al, "Physiological effects and mode of action of ZnO nanoparticles against postharvest fungal contaminants", Food Research International 101 (2017), Pages 274–279. http://dx.doi.org/10.1016/j.foodres.2017.08.019 The whole document | 1-24 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 01 February 2021 (01.02.2021) | 05 February 2021 (05.02.2021) |

| Name and mailing address of the ISA/ INAPI, Av. Libertador Bernardo O'Higgins 194, Piso 17, Santiago, Chile | Authorized officer BOBADILLA RABOY, Andres |
|---|---|
| Facsimile No. | Telephone No.   56-2-28870551 |

Form PCT/ISA/210 (second sheet) (January 2015)

**INTERNATIONAL SEARCH REPORT**
Information on patent family members

| International application No. |
| --- |
| PCT/IB2020/060115 |

| WO2014126584 A1 | 21-08-2014 | CN105121704 (A) | 02-12-2015 |
| | | EP2956570 (A1) | 23-12-2015 |
| | | US2015366213 (A1) | 24-12-2015 |
| | | US2019289854 (A1) | 26-09-2019 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8404263 B
- US 20100016443 A
- EP 0496106 A
- MX 2017012740

**Non-patent literature cited in the description**

- **TIMOTHY R. COOK et al.** Solar Energy Supply and Storage for the Legacy and Nonlegacy Worlds. *Chem. Rev.,* 2010, vol. 110, 6474-6502
- **ULRIKE G.** Can we improve global food security? A socio-economic and political perspective. *Food Sec.,* 2014, vol. 6, 187-200
- **SUKUMAR C. et al.** Climate change, plant diseases and food security: an overview. *Plant Pathology.,* 2011, vol. 60, 2-14
- **FABRICIO R. et al.** Silicon and Plan Diseases. Springer, 2015
- **GUNTZER F.** Benefits of plant silicon for crops: a review. *Agron Sustain Dev.,* 2012, vol. 32, 201-213
- **ALESSANDRO F. ; FABRICIO R. et al.** Silicon and Plan Diseases. Springer, 2015
- **CH. BAEHR et al.** Soluble Silicates-Highly Versatile and Safe. *International Journal for Applied Science,* 2007, vol. 133, 88-94
- **YANPING X et al.** Antibacterial Activity and Mechanism of Action of Zinc Oxide Nanoparticles against Campylobacter jejuni. *Journal of American Society for Microbiology,* 2011, vol. 77 (7), 2325-2331
- **RAJVEER S et al.** Synthesis, characterization and enhanced antimicrobial activity of reduced graphene oxide-zinc oxide nanocomposite. *Mater. Res. Express,* 2017, 1-8
- **LILI HE. ; YANG LIU. ; AZLIN MUSTAPHA. ; MENGSHI LIN.** *Microbiological Research.,* 2011, vol. 166, 207-215
- **BECHERI A. et al.** Synthesis and characterization of zinc oxide nanoparticles: application to textiles as UV-absorbers. *J. Nanopart. Res,* vol. 10, 679-689
- **AZAM A et al.** Low temperature synthesis of ZnO nanoparticles using mechanochemical route: a green chemistry approach. *IJTAS,* 2009, vol. 1 (2), 12-14